Europäisches Patentamt

(19) European Patent Office

Office européen des brevets

(11) Veröffentlichungsnummer: **0 124 010**
**B1**

(12) **EUROPÄISCHE PATENTSCHRIFT**

(45) Veröffentlichungstag der Patentschrift:
27.12.85

(51) Int. Cl.⁴: **C 07 C 85/11,** F 22 B 1/18,
F 22 B 23/02

(21) Anmeldenummer: 84104329.2

(22) Anmeldetag: 17.04.84

(54) **Verfahren zur kontinuierlichen Herstellung von aromatischen Diaminen unter gleichzeitiger Erzeugung von Dampf.**

(30) Priorität: 27.04.83 DE 3315191

(43) Veröffentlichungstag der Anmeldung:
07.11.84 Patentblatt 84/45

(45) Bekanntmachung des Hinweises auf die Patenterteilung:
27.12.85 Patentblatt 85/52

(84) Benannte Vertragsstaaten:
BE CH DE FR GB IT LI

(56) Entgegenhaltungen:
DE - A - 2 723 647
FR - A - 1 112 653
US - A - 2 432 099
US - A - 3 085 626

REVUE GENERALE DE THERMIQUE, Band 6, Nr. 65, Mai 1967, Seiten 671-696; E. BREARD: "Conception et technologie des différents types de chaudières à vapeur de puissance inférieure ou égale à 40 tonnes par heure" ULLMANN (4 erweiterte Auflage), Band 7, S 398-401.

(73) Patentinhaber: BAYER AG, Konzernverwaltung RP
Patentabteilung, D-5090 Leverkusen 1 Bayerwerk (DE)

(72) Erfinder: Becher, Dieter, Dr., Moltkestrasse 8,
D-4047 Dormagen (DE)
Erfinder: Witt, Harro, Dr., Möhlenbag 2, D-2224 Kuden (DE)
Erfinder: Dallmeyer, Hermann, Dr.,
Katterbachstrasse 106a, D-5060 Bergisch-Gladbach (DE)
Erfinder: Humburger, Siegbert, Dr.,
Carl-Rumpff-Strasse 8, D-5090 Leverkusen 1 (DE)
Erfinder: Schneider, Wolfgang, Dr., Waldblick 10,
D-5068 Odenthal (DE)
Erfinder: Stein, Harald, D.I., Niedersorpe 12,
D-5948 Schmalenberg 2 (DE)

## Beschreibung

Die Erfindung betrifft ein neuartiges Verfahren zur kontinuierlichen Herstellung von aromatischen Diaminen unter gleichzeitiger Erzeugung von Dampf durch katalytische Hydrierung der den Diaminen entsprechenden aromatischen Dinitroverbindungen unter Verwendung einer, mit Fieldrohren versehenen Blasensäule, wobei das durch die Fieldrohre zur Kühlung des Systems geleitete Wasser teilweise in Dampf überführt wird.

Verfahren zur Herstellung von aromatischen Aminen durch katalytische Hydrierung der ihnen zugrundeliegenden Nitroverbindungen sind in großer Zahl bekannt geworden (vgl. z. B. DE-OS 1 542 544, 1 947 851, 2 106 644, 2 135 154, 2 214 056, 2 456 308, BE-PS 631 964, 661 047, 661 946, FR-PS 1 359 438 oder GB-PS 768 111).

Bei der Umsetzung von aromatischen Dinitroverbindungen mit Wasserstoff wird eine beträchtliche Wärmemenge frei. Es hat daher nicht an Versuchen gefehlt, die freiwerdende Hydrierwärme bei der Herstellung von aromatischen Diaminen durch Hydrierung der entsprechenden Dinitroverbindungen zu nutzen. So kann z. B. das erwärmte Kühlwasser zur Beheizung von Räumen oder zur Erwärmung von Produktströmen oder auch zur Verdampfung niedrig siedender Lösungsmittel genutzt werden. Es ist bisher jedoch noch nicht gelungen, die Reaktionswärme zur Erzeugung von Dampf von mehr als 1 bar Überdruck zu nutzen. Dies ist insbesondere auf den Umstand zurückzuführen, daß bei der Hydrierung von aromatischen Dinitroverbindungen die Gefahr unkontrollierter Nebenreaktionen besteht. Diese können im harmloseren Fall zur Bildung von unerwünschten Nebenprodukten und damit zu Ausbeuteminderungen führen. Beispielhaft erwähnt seien in diesem Zusammenhang Kernhydrierungen, hydrogenolytische Spaltungen oder die Bildung von hochmolekularen, teerartigen Produkten. Im schlimmeren Falle können explosionsartige Reaktionen ablaufen. Eine weitere Schwierigkeit ist in dem Umstand zu sehen, daß der Partialdruck des Wasserstoffs bei hohen Temperaturen aufgrund der erhöhten Partialdrücke des Reaktionswassers und gegebenenfalls der mitverwendeten Lösungsmittel sinkt, ein unerwünschter Effekt, der ebenfalls Nebenreaktionen begünstigt. Weitere Schwierigkeiten bestehen in der oft unzureichenden Verteilung des Wasserstoffs im Reaktionsgemisch und in der Belegung der Kühlflächen mit harzartigen Verbindungen und/oder Katalysatoranteilen. Um diese unerwünschte Begleiteffekte soweit wie möglich auszuschließen, wurde bislang die großtechnische Hydrierung von aromatischen Dinitroverbindungen bei möglichst niedrigen Temperaturen durchgeführt, was natürlich der Erzeugung von Dampf mit einem über 1 bar liegenden Überdruck entgegen steht.

Es war daher die der Erfindung zugrundeliegende Aufgabe, ein neuartiges Verfahren zur Herstellung von aromatischen Diaminen durch Hydrierung von aromatischen Dinitroverbindungen zur Verfügung zu stellen, welches die Erzeugung von Dampf mit einem über 1 bar liegenden Überdruck gestattet, und welches dennoch nicht mit den genannten Nachteilen behaftet ist.

Diese Aufgabe konnte durch die Bereitstellung des nachstehend näher beschriebenen erfindungsgemäßen Verfahrens gelöst werden.

Gegenstand der Erfindung ist ein Verfahren zur kontinuierlichen Herstellung von aromatischen Diaminen unter gleichzeitiger Erzeugung von Dampf durch katalytische Hydrierung von aromatischen Dinitroverbindungen durch Umsetzung einer, im wesentlichen aus aromatischen Dinitroverbindungen, diesen Dinitroverbindungen entsprechenden aromatischen Diaminen, feinteilig suspendiertem, festem Hydrierungskatalysator, Wasser und organischem Lösungsmittel bestehenden, Reaktionssuspension mit überschüssigem Wasserstoff unter gleichzeitigem Einleiten der Reaktionssuspension und des Wasserstoffs in den unteren Teil eines Reaktors und kontinuierlicher Entnahme von Reaktionsendprodukt und überschüssigem Wasserstoff im oberen Teil des Reaktors, dadurch gekennzeichnet, daß man

a) als Reaktor eine mit Field-Rohren (2) versehene Blasensäule (1) verwendet, wobei die Kühlung des Reaktors mittels Wasser erfolgt, welches durch die Field-Rohre und einen Dampfabscheider (3) im Kreislauf geführt wird und welches zu 10 bis 50%, bezogen auf die Gesamtmenge des in die Field-Rohre eingespeisten Wassers, in den Field-Rohren in Dampf von mehr als 1 bar Überdruck überführt wird, wobei dem Wasserkreislauf an einer beliebigen Stelle hinter dem Abscheider und vor dem Eingang in die Fieldrohre eine, dem Dampf entsprechende Wassermenge zugeführt wird,

b) ein Verhältnis der Kühlfläche der Field-Rohre zum Reaktionsvolumen von 40 bis 400 m²/m³ einhält,

c) in der Blasensäule einen Überdruck von 40 bis 200 bar aufrechterhält, und man schließlich

d) die Menge der in die Blasensäule eingespeisten Reaktionssuspension einerseits und den Druck und damit die Temperatur des siedenden Kühlwassers andererseits so bemißt, daß in der Blasensäule eine Reaktionstemperatur zwischen 140 und 250° C vorliegt.

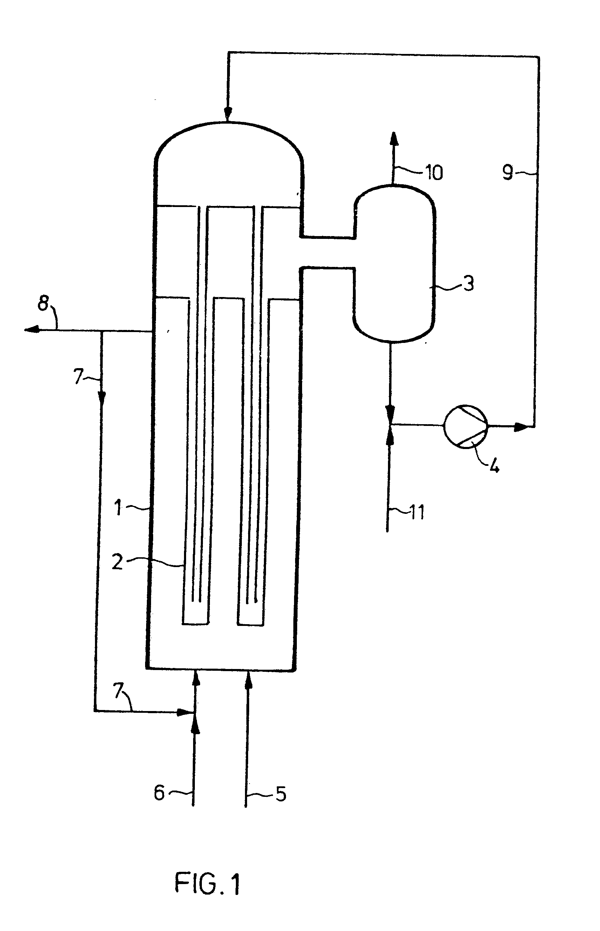

Die beim erfindungsgemäßen Verfahren einzusetzende Apparatur ist in der Zeichnung schematisch dargestellt.

In dieser Zeichnung bedeuten

(1) eine Blasensäule;
(2) in die Blasensäule hineinreichende Fieldrohre (Kühlfinger in Form doppelwandiger Rohre);

(3) ein Dampfabscheider;
(4) eine Pumpe zur Aufrechterhaltung des Kühlwasserkreislaufes;
(5) ein in den unteren Teil der Blasensäule mündendes Einleitungsrohr für die Reaktionssuspension;
(6) ein in den unteren Teil der Blasensäule einmündendes Einleitungsrohr für Wasserstoff;
(7) ein Rohrsystem für die Kreislaufführung des überschüssigen Wasserstoffs;
(8) ein Ableitungsrohr zur Entnahme von Fertigproduktsuspension;
(9) eine Rohrleitung zur Aufrechterhaltung des Kühlwasserkreislaufes;
(10) die Dampfentnahmestelle und
(11) die Frischwasserzugabe zum Kühlwasserkreislauf.

Bei der Durchführung des erfindungsgemäßen Verfahrens wird die in der Zeichnung schematisch dargestellte Apparatur verwendet, was jedoch nicht bedeuten soll, daß nicht auch solche Apparaturen verwendet werden können, die in unwesentlichen Punkten von der nur beispielhaft gedachten Darstellung abweichen. So muß z. B. die Einleitung des Wasserstoffs bzw. der Reaktionssuspension nicht unbedingt am Boden der Blasensäule erfolgen, sondern kann auch an einer beliebigen anderen Stelle im unteren Teil der Blasensäule vorgenommen werden. Denkbar ist auch eine Vereinigung des Wasserstoffs mit der Reaktionssuspension kurz vor Eintritt in die Blasensäule. Selbstverständlich kann die Blasensäule auch mit mehr als 2, parallel geschalteten, Fieldrohren versehen sein. Vorzugsweise werden Apparaturen verwendet, die 10 bis 200 parallel geschaltete Fieldrohre aufweisen. Wesentlich zur Durchführung des erfindungsgemäßen Verfahrens ist lediglich, daß das Verhältnis der Kühlfläche der Fieldrohre zum Reaktionsvolumen zwischen 40 und 400, vorzugsweise zwischen 50 und 150 m²/m³ liegt. Im allgemeinen weist die Blasensäule ein Volumen von 0,1 bis 10 m³ auf.

Bei der in die Blasensäule eingeleiteten Reaktionssuspension handelt es sich vorzugsweise um ein Gemisch, welches im wesentlichen aus

10 bis 40, vorzugsweise 25 bis 36 Gew.-Teilen aromatischen Dinitroverbindungen,
5 bis 20, vorzugsweise 7 bis 15 Gew.-Teilen an, den Dinitroverbindungen entsprechenden aromatischen Diaminen,
1,5 bis 10, vorzugsweise 2 bis 8 Gew.-Teilen an, in feiner Verteilung suspendiert vorliegendem, festem Katalysator,
5 bis 20, vorzugsweise 10 bis 15 Gew.-Teilen Wasser und
25 bis 60, vorzugsweise 35 bis 50 Gew.-Teilen Lösungsmittel

besteht.

Die Menge dieser, in die Blasensäule eingeleiteten Reaktionssuspension entspricht im allgemeinen einer Menge an, in der Reaktionssuspension vorliegender aromatischer Dinitroverbindung pro m³ Reaktionsvolumen von 2 bis 5, vorzugsweise 2,7 bis 4,5 t/h. Die Temperatur der Reaktionssuspension am Reaktoreintritt liegt im allgemeinen bei ca. 20 bis 80, vorzugsweise ca. 40 bis 60°C.

Die den Reaktor verlassende Fertigproduktsuspension besteht im wesentlichen aus aromatischem Diamin, Katalysator, Wasser und Lösungsmittel, d. h. die Ausbeute der Hydrierungsreaktion, bezogen auf eingesetzte Dinitroverbindung, ist nahezu quantitativ (höher als 95% der Theorie).

Die Fertigproduktsuspension kann nach Abtrennung des im Kreislauf geführten Wasserstoffs und Entspannen, beispielsweise durch Filtrieren vom suspendierten Katalysator, befreit und anschließend destillativ aufgearbeitet werden. Der wiedergewonnene Katalysator wird zweckmäßigerweise im Kreislauf an den Prozeßanfang zurückgeführt. Zur Herstellung der Reaktionssuspension kann gewünschtenfalls ein Teil der Flüssigphase der Fertigproduktsuspension vor oder nach ihrer destillativen Aufarbeitung wiederverwendet werden.

Die Kühlung des Systems erfolgt mittels über die Fieldrohre (2) und den Abscheider (3) im Kreislauf gepumpten Wassers, welches am Eingang zu den Fieldrohren eine Temperatur von ca. 70 bis 165°C aufweist und welche in den Fieldrohren zu 10 bis 50% in Dampf von mehr als 1 bar, vorzugsweise 1,5 bis 6 bar Überdruck überführt wird. Gleichzeitig wird dem Kühlwasserkreislauf an einer beliebigen Stelle hinter dem Abscheider und vor dem Eingang in die Fieldrohre eine, der Dampfmenge entsprechende, Wassermenge zugeführt.

Beim erfindungsgemäßen Verfahren können beliebige aromatische Dinitroverbindungen eingesetzt werden. Beispiele geeigneter Dinitroverbindungen sind 1,3-Dinitrobenzol, 2,4-Dinitrotoluol, 2,6-Dinitrotoluol, im wesentlichen aus den beiden letztgenannten Isomeren bestehende, technische Dinitrotoluol-Gemische oder 1,5-Dinitronaphthalin. Besonders bevorzugt werden als aromatische Dinitroverbindungen 2,4-Dinitrotoluol oder dessen technische Gemische mit bis zu 35 Gew.-%, bezogen auf Gesamtgemisch, an 2,6-Dinitrotoluol eingesetzt. Diese technischen Gemische können auch untergeordnete Mengen, d. h. insgesamt bis max. 5 Gew.-%, bezogen auf Gesamtgemisch, an 2,3-, 2,5- oder 3,4-Dinitrotoluol enthalten.

Für das erfindungsgemäße Verfahren werden die an sich bekannten Hydrierungskatalysatoren für aromatische Nitroverbindungen verwendet. Gut geeignet sind die Metalle der 8. Nebengruppe des Periodensystems der Elemente, die beispielsweise auf Trägermaterialien wie Oxiden von Magnesium, Aluminium und/oder Silicium aufgebracht sein können. Vorzugsweise werden Raneyeisen, -kobalt und/oder -nickel, insbesondere Raneynickel, verwendet. Die Katalysatoren werden in feinverteiltem Zustand eingesetzt und liegen in der Reaktionssuspension feinteiligsuspendiert vor. Die Aufrechterhaltung dieser Suspension bei der Durchführung des erfindungsgemäßen Verfahrens wird durch die Turbulenz, hervorgerufen durch den Wasserstoffstrom, gewährleistet.

Für das erfindungsgemäße Verfahren geeignete Lösungsmittel sind insbesondere gesättigte, einwertige, aliphatische Alkohole mit 1 bis 6 Kohlenstoffatomen wie z. B. Methanol, Ethanol, Isopropanol oder n-Hexanol. Besonders bevorzugt ist Isopropanol.

Der Wasserstoff wird, bezogen auf die Dinitroverbindung, in hohem Überschuß eingesetzt und wird unter Aufrechterhaltung eines Wasserstoffdrucks von 40 bis 200 bar, vorzugsweise 70 bis 120 bar, in den Reaktor eingeleitet.

Das erfindungsgemäße Verfahren gestattet die Herstellung von aromatischen Diaminen in einer Raum/Zeit-Ausbeute pro m³ Reaktionsvolumen von 1,4 bis 3,5 t/h, vorzugsweise 1,8 bis 3 t/h unter gleichzeitiger Erzeugung von Dampf eines über 1 bar, vorzugsweise bei 1,5 bis 6 bar, liegenden Überdrucks in einer Menge von ca. 1 bis 2,5 t pro Tonne an eingesetzter Dinitroverbindung.

### Beispiel

Der in dem Beispiel eingesetzte Reaktor entspricht der schematischen Darstellung der Zeichnung. Das Reaktionsvolumen der Blasensäule beträgt ca. 0,22 m³. Der Reaktor ist mit 37 parallel geschalteten Fieldrohren versehen, die insgesamt einer Kühlfläche von ca. 18 m² entsprechen. Die Menge des in die Fieldrohre eingespeisten Kühlwassers beträgt ca. 15 m³/h, die Temperatur des in die Fieldrohre eingespeisten Wassers liegt bei ca. 151° C.

Mit einer Hochdruckpumpe werden 3 t/h einer 55° C warmen Mischung, bestehend aus 33 Gew.-Teilen Dinitrotoluol (2,4/2,6-Isomerengemisch im Gewichtsverhältnis 80 : 20), 9 Gew.-Teilen eines entsprechenden Diaminotoluol-Gemisches, 11 Gew.-Teilen Wasser, 42 Gew.-Teilen Isopropanol und 5 Gew.-Teilen feinteiligen Raney-Nickels, in die Blasensäule (1) gefördert. Durch gleichzeitiges Einleiten von Wasserstoff wird ein Druck im Reaktor von 100 bar aufrechterhalten. Die max. Reaktionstemperatur im unteren Drittel des Reaktors liegt bei 220° C. Dem Reaktor wird gleichzeitig kontinuierlich eine Fertigproduktsuspension entnommen, deren Menge und Diamingehalt einer Ausbeute an Diamin von ca. 98%, bezogen auf eingesetztes Dinitrotoluol, entspricht. Gleichzeitig wird dem System über (10) 1,75 t/h Dampf eines Überdrucks von 4 bar entnommen. Das der Dampfmenge entsprechende Wasser wird dem System kontinuierlich über (11) zugeführt.

### Patentansprüche

1. Verfahren zur kontinuierlichen Herstellung von aromatischen Diaminen unter gleichzeitiger Erzeugung von Dampf durch katalytische Hydrierung von aromatischen Dinitroverbindungen durch Umsetzung einer, im wesentlichen aus aromatischen Dinitroverbindungen, diesen Dinitroverbindungen entsprechenden aromatischen Diaminen, feinteilig suspendiertem, festem Hydrierungskatalysator, Wasser und organischem Lösungsmittel bestehenden, Reaktionssuspension mit überschüssigem Wasserstoff unter gleichzeitigem Einleiten der Reaktionssuspension und des Wasserstoffs in den unteren Teil eines Reaktors und kontinuierlicher Entnahme von Reaktionsendprodukt und überschüssigem Wasserstoff im oberen Teil des Reaktors, dadurch gekennzeichnet, daß man

a) als Reaktor eine mit Fieldrohren (2) versehene Blasensäule (1) verwendet, wobei die Kühlung des Reaktors mittels Wasser erfolgt, welches durch die Fieldrohre und einen Dampfabscheider (3) im Kreislauf geführt wird und welches zu 10 bis 50%, bezogen auf die Gesamtmenge des in die Fieldrohre eingespeisten Wassers, in den Fieldrohren in Dampf von mehr als 1 bar Überdruck überführt wird, wobei dem Wasserkreislauf an einer beliebigen Stelle hinter dem Abscheider und vor dem Eingang in die Fieldrohre eine, dem Dampf entsprechende Wassermenge zugeführt wird,

b) ein Verhältnis der Kühlfläche der Fieldrohre zum Reaktionsvolumen von 40 bis 400 m²/m³ einhält,

c) in der Blasensäule einen Überdruck von 40 bis 200 bar aufrechterhält, und man schließlich

d) die Menge der in die Blasensäule eingespeisten Reaktionssuspension einerseits und den Druck und damit die Temperatur des siedenden Kühlwassers andererseits so bemißt, daß in der Blasensäule eine Reaktionstemperatur zwischen 140 und 250° C vorliegt.

2. Verfahren gemäß Anspruch 1, dadurch gekennzeichnet, daß man eine Reaktionssuspension verwendet, welche im wesentlichen aus

10 bis 40 Gew.-Teilen Dinitroverbindungen,
5 bis 20 Gew.-Teilen Diamin,
1,5 bis 10 Gew.-Teilen Hydrierungskatalysator,
25 bis 60 Gew.-Teilen Lösungsmittel und
5 bis 20 Gew.-Teilen Wasser

besteht.

3. Verfahren gemäß Ansprüchen 1 und 2, dadurch gekennzeichnet, daß man als aromatische Dinitroverbindungen 2,4-Dinitrotoluol oder dessen technische Gemische mit 2,6-Dinitrotoluol verwendet.

4. Verfahren gemäß Ansprüchen 1 bis 3, dadurch gekennzeichnet, daß man als Katalysator Raneyeisen, -kobalt und/oder -nickel verwendet.

5. Verfahren gemäß Ansprüchen 1 bis 4, dadurch gekennzeichnet, daß man als Lösungsmittel einen gesättigten, einwertigen, aliphatischen Alkohol mit 1 bis 6 Kohlenstoffatomen verwendet.

6. Verfahren gemäß Ansprüchen 1 und 2, dadurch gekennzeichnet, daß die Raum/Zeit-Ausbeute pro Kubikmeter Reaktionsvolumen und Stunde zwischen 1,4 und 3,5 t Diamin liegt.


## Claims

1. Process for the continuous production of aromatic diamines with the simultaneous production of steam by catalytic hydrogenation of aromatic dinitro compounds by reacting a reaction suspension consisting essentially of aromatic dinitro compounds, aromatic diamines corresponding to these dinitro compounds, a finely-divided suspended solid hydrogenation catalyst, water and organic solvent, with excess hydrogen, with simultaneous introduction of the reaction suspension and the hydrogen into the lower part of a reactor and/with the continuous removal of reaction end product and excess hydrogen from the upper part of the reactor, characterised in that

a) a bubble column (1) provided with field tubes (2) is used as the reactor, the cooling of the reactor being carried out by means of water which is circulated through the field tubes and a steam separator (3) and 10 to 50% of which, based on the total quantity of water introduced into the field tubes, is converted in the field tubes into steam of an excess pressure of more than 1 bar, a quantity of water corresponding to the steam being supplied to the water cycle at any desired point downstream of the separator and upstream of the inlet into the field tubes,

b) a ratio of the cooling area of the field tubes to the reaction volume of 40 to 400 m$^2$/m$^3$ is observed,

c) an excess pressure of 40 to 200 bar is maintained in the bubble column, and finally

d) the quantity of the reaction suspension introduced into the bubble column on the one hand the pressure and thus the temperature of the boiling cooling water on the other hand are adjusted in such a manner that a reaction temperature of between 140 and 250° C is present in the bubble column.

2. Process according to Claim 1, characterised in that a reaction suspension is used which essentially consists of

10 to 40  parts by weight of dinitro compounds,
 5 to 20  parts by weight of diamine,
1.5 to 10  parts by weight of hydrogenation catalyst,
25 to 60  parts by weight of solvent and
 5 to 20  parts by weight of water.

3. Process according to Claims 1 and 2, characterised in that 2,4-dinitrotoluene or commercial mixtures thereof with 2,6-dinitrotoluene are used as the aromatic dinitro compounds.

4. Process according to Claims 1 to 3, characterised in that Raney iron, Raney cobalt and/or Raney nickel are used as the catalyst.

5. Process according to Claims 1 to 4, characterised in that a saturated, monohydric, aliphatic alcohol with 1 to 6 carbon atoms is used as the solvent.

6. Process according to Claims 1 and 2, characterised in that the space/time yield per cubic metre of reaction volume and per hour is between 1.4 and 3.5 t of diamine.


## Revendications

1. Procédé pour la préparation continue de diamines aromatiques avec production simultanée de vapeur par hydrogénation catalytique de composés aromatiques dinitrés par réaction d'une suspension de réaction consistant essentiellement en composés aromatiques dinitrés, diamines aromatiques correspondant à ces composés dinitrés, un catalyseur d'hydrogénation solide à l'état de fines particules en suspension, de l'eau et un solvant organique, à l'aide d'un excès d'hydrogène, par injection simultanée de la suspension de réaction et de l'hydrogène dans la partie inférieure d'un réacteur et évacuation continue du produit final de réaction et de l'excès d'hyrogène dans la partie supérieure du réacteur, le procédé se caractérisant en ce que

a) on utilise en tant que réacteur une colonne à bulles (1) équipée de tubes Field (2), la réfrigération du réacteur étant assurée à l'aide d'eau circulant au travers des tubes Field et d'un séparateur de vapeur (3) et qui est convertie en proportions de 10 à 50%, par rapport à la quantité totale de l'eau envoyée dans les tubes Field, dans ceux-ci, en vapeur à une pression manométrique supérieure à 1 bar, avec apport, dans le circuit d'eau, à un endroit quelconque en aval du séparateur, et en amont de l'entrée dans les tubes Field, d'une quantité d'eau correspondant à la quantité de vapeur,

b) on maintient un rapport de 40 à 400 m$^2$/m$^3$ entre la surface de refroidissement des tubes Field et le volume de réaction,

c) on maintient dans la colonne à bulles une pression manométrique de 40 à 200 bar et, finalement,

d) on règle la quantité de la suspension de réaction envoyée dans la colonne à bulles, d'une part, et la pression et par conséquent la température de l'eau de réfrigération bouillante, d'autre part, de manière que la température de réaction dans la colonne à bulles se situe entre 140 et 250° C.

2. Procédé selon la revendication 1, caractérisé en ce que l'on utilise une suspension de réaction qui consiste essentiellement en

| | | |
|---|---|---|
| 10 | à 40 | parties en poids de composés dinitrés, |
| 5 | à 20 | parties en poids de diamine, |
| 1,5 | à 10 | parties en poids de catalyseur d'hydrogénation, |
| 25 | à 60 | parties en poids de solvant et |
| 5 | à 20 | parties en poids d'eau. |

3. Procédé selon les revendications 1 et 2, caractérisé en ce que l'on utilise en tant que composés aromatiques dinitrés le 2,4-dinitrotoluène ou ses mélanges industriels avec le 2,6-dinitrotoluène.

4. Procédé selon les revendications 1 à 3, caractérisé en ce que l'on utilise en tant que catalyseur du fer, du cobalt et/ou du nickel de Raney.

5. Procédé selon les revendications 1 à 4, caractérisé en ce que l'on utilise en tant que solvant un alcool aliphatique monovalent saturé contenant 1 à 6 atomes de carbone.

6. Procédé selon les revendications 1 à 2, caractérisé en ce que le rendement spatial horaire par $m^3$ de volume de réaction et par heure se situe entre 1,4 et 3,5 tonnes de diamine.

FIG. 1